# FASCICULE DE BREVET EUROPEEN

(11) **EP 4 019 083 B1**
(45) Date de publication et mention de la délivrance du brevet: **22.02.2023**
(21) Numéro de dépôt: 20315501.5
(22) Date de dépôt: 22.12.2020
(51) Int. Cl.: A61N 1/372, A61F 2/00, A61N 1/375, A61N 1/378

(54) **ACCESSOIRE POUR LE TRANSPORT ET LE STOCKAGE D'UN IMPLANT CARDIAQUE AUTONOME DE TYPE CAPSULE LEADLESS**
ZUBEHÖR FÜR DEN TRANSPORT UND DIE LAGERUNG EINES AUTONOMEN HERZIMPLANTATS VOM TYP LEITUNGSLOSE KAPSEL
ACCESSORY FOR TRANSPORTING AND STORING A LEADLESS CAPSULE TYPE AUTONOMOUS CARDIAC IMPLANT

(43) Date de publication de la demande: 29.06.2022
(73) Titulaire: Cairdac, 92160 Antony (FR)
(72) Inventeur: Makdissi, Alaa, 75011 Paris (FR); Regnier, Willy, 91160 Longjumeau (FR); Nguyen-Dinh, An, 37520 La Riche (FR)
(74) Mandataire: Dupuis-Latour, Dominique

(56) Documents cités:
- EP-A1- 3 708 221
- WO-A1-98/08567
- US-A1- 2003 204 218
- US-A1- 2005 075 695
- US-A1- 2008 103 557
- US-B1- 6 181 105

## Description

### CONTEXTE DE L'INVENTION

### Domaine technique

L'invention concerne les dispositifs médicaux durant leur transport et leur stockage, pendant la période comprise entre leur fabrication et le moment où ils sont utilisés par le praticien afin d'être implantés chez un patient.

Elle concerne plus précisément ceux de ces dispositifs qui incorporent un système d'auto-alimentation comprenant un récupérateur d'énergie mécanique, également dénommé "harvester" ou "scavenger" associé à un organe de stockage d'énergie intégré tel qu'une micro-batterie tampon rechargeable ou une capacité à hautes performances.

Le récupérateur peut être notamment du type dit "PEH" (*Piezoelectric Energy Harvester*), qui utilise comme transducteur mécanoélectrique une lame piézoélectrique oscillante couplée à une masse mobile inertielle.

Le transport et le stockage de tels dispositifs implique, comme on l'expliquera plus bas, un certain nombre de contraintes et difficultés spécifiques, propres à la présence du harvester, et qui ne se rencontrent pas avec les dispositifs médicaux conventionnels alimentés par une pile statique de très longue durée de vie (dispositifs tels que stimulateurs cardiaques, défibrillateurs implantables, etc.).

### État de la technique antérieure

Les récupérateurs de type "harvester" sont notamment utilisés pour alimenter des dispositifs médicaux autonomes implantables (ci-après "implants"), en particulier des capsules autonomes destinées à être implantées dans une cavité cardiaque.

L'invention n'est toutefois pas limitée à un tel dispositif, elle est applicable aussi bien à de nombreux autres types de dispositifs médicaux implantables miniaturisés, quelle que soit leur destination fonctionnelle, cardiaque ou autre. L'un des aspects critiques de ces dispositifs miniaturisés est celui de l'autonomie électrique. En effet, la durée de vie d'un tel dispositif étant d'environ 8 à 10 ans, compte tenu des très faibles dimensions il n'est pas possible d'utiliser une batterie conventionnelle, même à forte densité. Un harvester pallie cet inconvénient, en recueillant l'énergie mécanique résultant des mouvements divers subis par le corps de l'implant. Ces mouvements peuvent avoir pour origine un certain nombre de phénomènes se produisant notamment au rythme des battements cardiaques, tels que les secousses périodiques de la paroi sur laquelle est ancré l'implant, les vibrations des tissus cardiaques liées entre autres aux fermetures et ouvertures des valves cardiaques, ou encore les variations de débit du flux sanguin dans le milieu environnant, qui sollicitent l'implant et le font osciller au rythme des variations de débit. L'énergie mécanique recueillie est convertie au moyen d'un transducteur mécanoélectrique approprié en une énergie électrique (tension ou courant) suffisante pour assurer l'alimentation des divers circuits et capteurs du dispositif et la recharge de l'organe de stockage d'énergie. Ce système d'alimentation permet ainsi au dispositif de fonctionner en parfaite autonomie électrique pendant toute sa durée de vie.

Cette technique de récupération d'énergie est particulièrement bien adaptée à l'alimentation des capsules autonomes implantées dépourvues de toute liaison physique à un dispositif distant. Ces capsules sont dénommées pour cette raison "capsules leadless", pour les distinguer des électrodes ou capteurs disposés à l'extrémité distale d'une sonde (*lead*) parcourue sur toute sa longueur par un ou plusieurs conducteurs reliés à un générateur connecté à l'extrémité opposée, proximale.

Dans ce cas d'une application cardiaque, la capsule leadless surveille en continu le rythme du patient et délivre si nécessaire au coeur des impulsions électriques de stimulation, de resynchronisation et/ou de défibrillation en cas de troubles du rythme détecté par la capsule. La capsule leadless peut être une capsule épicardique, fixée à la paroi extérieure du coeur, ou bien une capsule endocavitaire, fixée à la paroi intérieure d'une cavité ventriculaire ou auriculaire, ou bien encore une capsule fixée sur une paroi d'un vaisseau à proximité du myocarde. La fixation de la capsule au site d'implantation est assurée par un système d'ancrage saillant prolongeant le corps de la capsule et destinée à pénétrer dans le tissu cardiaque, notamment au moyen d'une vis.

La capsule comprend divers circuits électroniques, capteurs, etc. ainsi que des moyens émetteurs/récepteurs de communication sans fil pour l'échange de données à distance, le tout étant intégré dans un corps de très petites dimensions qui puisse être implanté dans des sites difficilement accessibles ou laissant peu d'espace, tels que l'apex du ventricule, la paroi interne de l'oreillette, etc.

Les US 2009/0171408 A1 (Solem), US 2017/0151429 A1 (Regnier) et WO 2018/122244 A1 (Regnier) décrivent divers exemples de telles capsules leadless intracardiaques.

Le récupérateur d'énergie intégré à ces capsules peut notamment mettre en oeuvre un ensemble pendulaire inertiel soumis aux sollicitations externes décrites plus haut : une masse mobile (dite "masse sismique") couplée à un élément élastiquement déformable est entrainée au gré des mouvements de la capsule et vibre à une fréquence propre d'oscillation libre. L'énergie mécanique de l'oscillation est convertie en énergie électrique par un transducteur mécanoélectrique, en particulier par un composant tel qu'une lame piézoélectrique encastrée à l'une de ses extrémités et couplée à la masse inertielle à son autre extrémité, qui est libre. La lame, sollicitée de façon cyclique et alternative en flexion, engendre des charges électriques qui sont récupérées en surface du composant pour être utilisées par le système d'autoalimentation de la capsule leadless afin d'assurer l'alimentation des divers circuits électroniques et capteurs de la capsule, ainsi que la recharge de l'organe de stockage d'énergie.

Un tel récupérateur d'énergie de type PEH destiné à l'alimentation d'un implant à partir des oscillations d'une lame piézoélectrique est notamment décrit dans le US 3,456,134 A (Ko) et dans le WO 2019/001829 A1 (Cairdac).

Dans la phase préalable à l'implantation, c'est-à-dire pendant le transport et le stockage de l'implant, plusieurs difficultés peuvent apparaître.

Un *premier problème* réside dans la nécessité de maintenir pendant toute la durée du stockage (qui peut durer de nombreux mois) un niveau de charge de la micro-batterie tampon intégrée qui soit suffisant pour maintenir en veille les circuits électriques de l'implant jusqu'au moment de l'implantation, moment auquel ces circuits seront activés pour devenir pleinement fonctionnels.

La consommation de l'implant en veille est certes très faible, de l'ordre de 1 µA, incluant l'alimentation des circuits de veille et le courant d'autodécharge de l'organe de stockage d'énergie. Mais les organes de stockage d'énergie utilisés avec les implants miniaturisés, qu'il s'agisse de micro-batteries tampon rechargeables ou de condensateurs de forte capacité (dans la suite on utilisera le terme générique de "batterie") ont une capacité limitée, typiquement à une valeur de l'ordre de 1 mAh, ce qui assure une autonomie sur étagère d'environ 1000 h, soit approximativement 40 jours seulement - en effet, sur étagère l'implant est immobile et il n'y a donc pas de recharge par le harvester.

Ces chiffres sont à comparer à ceux des implants traditionnels (pacemakers, défibrillateurs implantables, etc.) munis d'une pile statique de longue durée, dont la capacité est généralement d'au moins 100 mAh : dans ce cas, pour un même courant de 1 µA, au bout de 20 mois de stockage la pile n'aura perdu que 15 % de sa capacité nominale.

Pour de tels implants conventionnels, le WO 98/08567 A1 (Pacesetter) propose de coupler à l'implant une source externe d'énergie permettant d'alimenter l'implant pendant toute la durée du stockage, préservant ainsi la pile interne d'alimentation de ce dernier. Un circuit d'interfaçage permet de commuter à volonté l'alimentation des circuits de l'implant soit sur la source externe, soit sur la pile interne.

Les US 6 181 105 B1 (Cutolo et al.), US 2008/103557 A1 (Davis et al.), US 2003/204218 A1 (Vogel et al.) et US 2005/075695 A1 (Schommer) divulguent d'autres systèmes d'alimentation ou de recharge d'un dispositif médical implantable à partir d'une source externe d'énergie.

Un *second problème,* propre aux implants munis d'un harvester, est celui de la fragilité du transducteur mécanoélectrique et des risques d'endommagement de celui-ci pendant le transport - problème qui ne se pose évidemment pas avec les implants munis d'une pile statique.

En particulier dans le cas d'un harvester avec un ensemble pendulaire à lame piézoélectrique, cet ensemble risque d'être sollicité pendant le transport à des accélérations bien supérieures à celles pour lesquelles il a été conçu pour fonctionner après implantation *in situ.* Il peut également subir des vibrations à des fréquences proches de la fréquence d'oscillation libre de l'ensemble masse-ressort de l'ensemble pendulaire, avec dans ce cas le risque d'une entrée en résonance engendrant des amplitudes et des contraintes excessives, délétères pour l'intégrité de la lame piézoélectrique.

Un autre but de l'invention est de protéger l'implant à l'égard de ce risque d'endommagement du harvester pendant le transport entre l'usine de fabrication et le lieu de stockage final.

Le US 2019/070422 A1 (Regnier) décrit une capsule leadless avant implantation, configurée pour son stockage et son transport.

Le problème envisagé par ce document est toutefois différent : il s'agit d'assurer dans cette situation un échange de données entre la capsule et l'environnement extérieur par une technique de communication par voie intracorporelle dite HBC (*Human Body Communication*), qui est une technique où la communication est conduite par un milieu constitué par les tissus ou fluides interstitiels du corps d'un patient et ne peut de ce fait être normalement mise en oeuvre qu'après implantation. Mais le problème de l'alimentation de la capsule n'est pas envisagé, celle-ci étant supposée être dans un état fonctionnel.

### RÉSUMÉ DE L'INVENTION

Pour résoudre les problèmes ci-dessus, l'invention propose un accessoire pour le transport et le stockage d'un implant comprenant les caractéristiques énoncées dans la revendication 1.

Les sous-revendications visent diverses formes de réalisation subsidiaires, avantageuses, de l'invention.

L'invention a également pour objet un conditionnement pour le transport et le stockage d'un implant tel que défini ci-dessus, ce conditionnement comprenant un emballage étanche définissant un volume interne stérile enfermant l'implant et comprenant un accessoire tel que défini ci-dessus.

La source externe peut être une pile électrique non rechargeable logée à l'intérieur de l'emballage étanche, ou un récepteur d'énergie inductive logé à l'intérieur de l'emballage étanche et couplé par voie non galvanique à un émetteur d'énergie inductive extérieur à l'emballage étanche.

### DESCRIPTION SOMMAIRE DES DESSINS

La Figure 1 illustre une capsule leadless dans son environnement, implantée au fond du ventricule droit du myocarde d'un patient.
La Figure 2 est une vue longitudinale générale d'une capsule leadless comprenant un récupérateur d'énergie à ensemble pendulaire.
La Figure 3 présente sous forme schématique les principaux blocs fonctionnels constitutifs d'une capsule leadless.
La Figure 4 est une vue générale illustrant le conditionnement complet, avec l'implant et ses accessoires enfermés dans un emballage étanche stérile.
La Figure 5 illustre plus précisément le support de protection et de calage de l'implant, en situation à l'intérieur de l'emballage étanche, pendant le transport le stockage.
La Figure 6 est une variante de la Figure 5, où la pile "bâton" a été remplacée par une pile "bouton".
La Figure 7 illustre, en perspective, l'implant placé à l'intérieur de l'une des deux pièces déformables constituant le support de protection et de calage.
La Figure 8 est une vue en élévation, partiellement en coupe, correspondant à la Figure 7.
La Figure 9 est une vue de bout correspondant à la Figure 7.
La Figure 10 est homologue de la Figure 7, avec la seule pièce déformable sans l'implant.
La Figure 11 est une vue en plan de la pièce déformable illustrée Figure 10.
La Figure 12 est une vue de détail, en élévation, de la partie avant de l'implant en appui contre une butée frontale du support de protection.
La Figure 13 est homologue de la Figure 12, en coupe transversale.
La Figure 14 est un schéma électrique expliquant la manière dont fonctionne le système de recharge de la batterie tampon de l'implant.

### DESCRIPTION DÉTAILLÉE

### DE MODES DE RÉALISATION PRÉFÉRENTIELS DE L'INVENTION

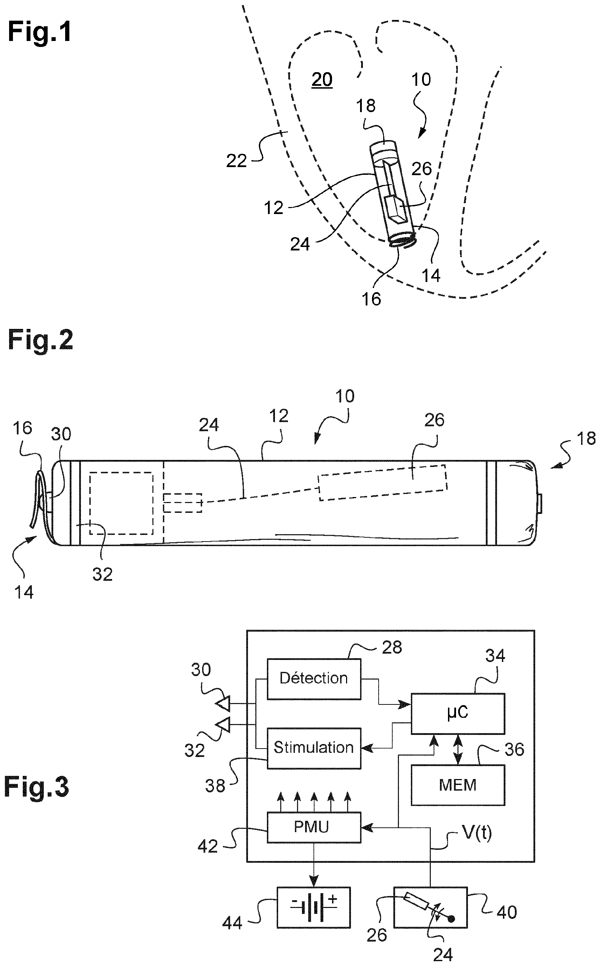

Sur les Figures 1 et 2 on a représenté un implant de type capsule leadless 10 dans une application à la stimulation cardiaque.

La capsule 10 se présente extérieurement sous la forme d'un corps tubulaire allongé cylindrique 12 enfermant les divers circuits électroniques et d'alimentation de la capsule ainsi qu'un récupérateur d'énergie à ensemble pendulaire. Les dimensions typiques d'une telle capsule sont un diamètre de l'ordre de 6 mm pour une longueur d'environ 25 à 40 mm.

Le corps tubulaire 12 comporte à son extrémité avant (distale) 14 un élément d'ancrage saillant, par exemple une vis hélicoïdale 16 pour maintenir la capsule sur le site d'implantation. L'extrémité opposée (proximale) 18 de la capsule 10 est une extrémité libre, qui est seulement pourvue de moyen de liaison temporaire à un cathéter-guide (non représenté) ou autre accessoire d'implantation pour la mise en place ou l'explantation de la capsule.

Dans l'exemple illustré Figure 1, la capsule leadless 10 est un implant endocavitaire implanté à l'intérieur d'une cavité 20 du myocarde 22, par exemple à l'apex du ventricule droit. En variante, toujours dans une application à la stimulation cardiaque, la capsule peut être également implantée sur le septum interventriculaire ou sur une paroi auriculaire, ou encore être une capsule épicardique placée sur une région externe du myocarde.

La capsule leadless 10 est dotée d'un module récupérateur d'énergie comprenant un ensemble pendulaire inertiel qui oscille à l'intérieur de la capsule au gré des diverses sollicitations externes auxquelles la capsule est soumise. Ces sollicitations peuvent notamment résulter : des mouvements de la paroi à laquelle est ancrée la capsule, qui sont transmis au corps tubulaire 12 par la vis d'ancrage 16 ; et/ou des variations du débit du flux sanguin dans le milieu environnant la capsule, qui produisent des oscillations du corps tubulaire 12 au rythme des battements cardiaques ; et/ou des vibrations diverses transmises par les tissus cardiaques. L'ensemble pendulaire peut en particulier être constitué d'une lame piézoélectrique 24 encastrée à l'une de ses extrémités et dont l'extrémité opposée, libre, est couplée à une masse inertielle mobile 26, l'ensemble formant un système pendulaire de type masse-ressort. Du fait de son inertie, la masse 26 soumet la lame 24 à une déformation de type vibratoire de part et d'autre d'une position neutre ou non déformée correspondant à une position stable de repos en l'absence de toute sollicitation. La lame piézoélectrique 24 assure en outre une fonction de transducteur mécanoélectrique permettant de convertir la contrainte mécanique de flexion qui lui est appliquée en charges électriques qui sont collectées pour produire un signal électrique qui, après redressement, stabilisation et filtrage alimentera les divers circuits électroniques de la capsule.

La Figure 3 est un synoptique des divers circuits électriques et électroniques intégrés à la capsule leadless, présentés sous forme de blocs fonctionnels.

Le bloc 28 désigne un circuit de détection de l'onde de dépolarisation cardiaque, qui est relié à une électrode de cathode 30 en contact avec le tissu cardiaque et à une électrode d'anode 32 associée, par exemple une électrode annulaire formée sur le corps tubulaire de la capsule (voir Figure 2). Le bloc de détection 28 comprend des filtres et des moyens de traitement analogique et/ou numérique du signal recueilli. Le signal ainsi traité est appliqué à l'entrée d'un microcalculateur 34 associé à une mémoire 36. L'ensemble électronique comporte également un circuit de stimulation 38 opérant sous le contrôle du microcalculateur 34 pour délivrer en tant que de besoin au système d'électrodes 30, 32 des impulsions de stimulation du myocarde.

Il est par ailleurs prévu un circuit récupérateur d'énergie PEH 40, constitué par l'ensemble pendulaire formé par la lame piézoélectrique 24 et la masse inertielle 30 décrits plus haut aux Figures 1 et 2. La lame piézoélectrique 24 assure aussi une fonction de transducteur mécanoélectrique qui convertit en charges électriques les sollicitations mécaniques subies et produit un signal électrique variable V(t), qui est un signal alternatif oscillant à la fréquence d'oscillation libre de l'ensemble pendulaire lame 24/masse 30 et au rythme des battements successifs du myocarde auquel la capsule est couplée.

Le signal électrique variable V(t) est délivré à un circuit de gestion de l'énergie ou PMU 42. Le PMU 42 redresse et régule le signal V(t) de manière à produire en sortie une tension ou un courant continus stabilisés servant à l'alimentation des divers circuits électroniques et à la recharge d'une micro-batterie tampon intégrée 44 (on assimilera au cas d'une micro-batterie celui d'un condensateur de forte capacité, qui remplit la même fonction de stockage temporaire d'une énergie électrique permettant d'assurer l'alimentation de l'ensemble des circuits de l'implant).

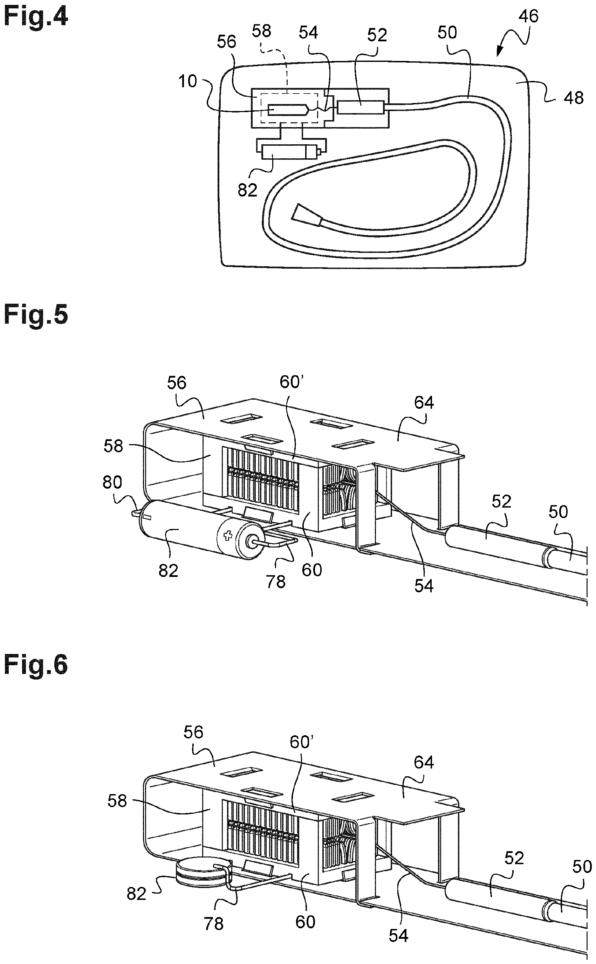

La Figure 4 est une vue générale illustrant le conditionnement complet, avec l'implant et ses accessoires enfermés dans un emballage étanche stérile.

Le conditionnement comprend avec un emballage étanche 46 définissant un volume interne étanche et stérile 48 dans lequel est enfermée la capsule 10. L'emballage contient également, outre la capsule, un cathéter 50 destiné à l'implantation, qui se termine côté distal (à proximité de la capsule) par un logement ou "housing" 52 recevant et protégeant la capsule pendant le guidage dans le réseau veineux et empêchant également que la vis d'ancrage 16 ne vienne blesser les parois vasculaires. Dans l'emballage, la capsule est sortie du housing 52 et n'est reliée au cathéter que par un fil de sécurité ou "fil d'Ariane" 54 dont elle ne sera libérée qu'une fois atteint le site d'implantation définitif.

La capsule 10 est disposée à l'intérieur d'un support de protection et de calage 56, incluant une structure d'amortissement 58 caractéristique de l'invention.

Les Figures 5 à 13 illustrent plus en détail la manière dont est réalisé ce support de protection 56.

Le support 56 comprend une structure d'amortissement 58 à l'intérieur de laquelle est logée la capsule 10 de manière à protéger celle-ci des chocs et à filtrer les vibrations éventuelles qu'elle pourrait subir, notamment à des fréquences proches de la fréquence de résonance libre de l'ensemble pendulaire du harvester, de manière à ne pas risquer d'endommager celui-ci par des amplitudes de déformation excessives ou des chocs pendant le transport.

La structure d'amortissement 58 est constituée de deux pièces déformables 60, 60' identiques, montées l'une contre l'autre de manière à prendre en sandwich la capsule 10 et caler celle-ci en position.

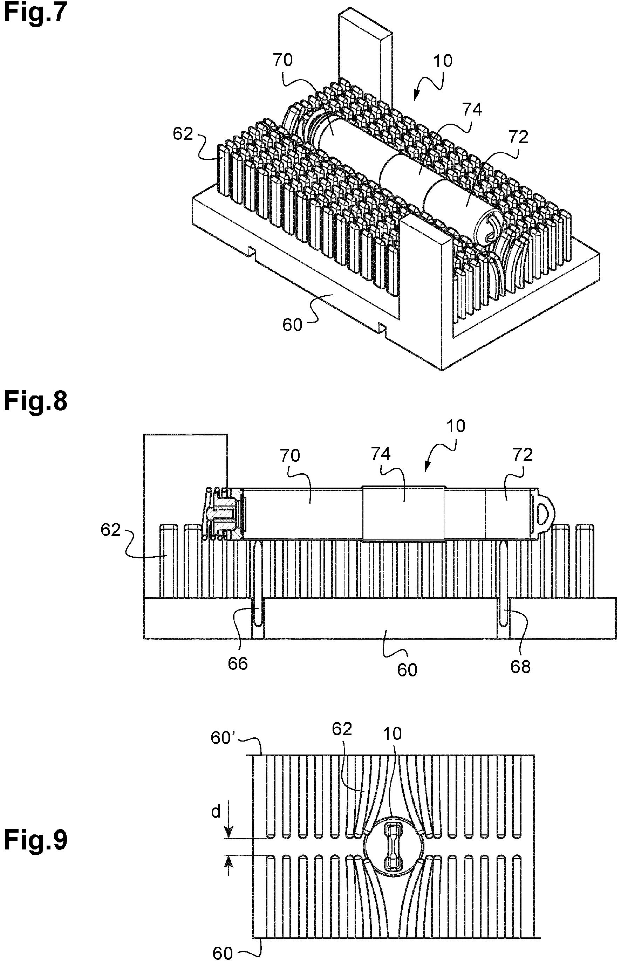

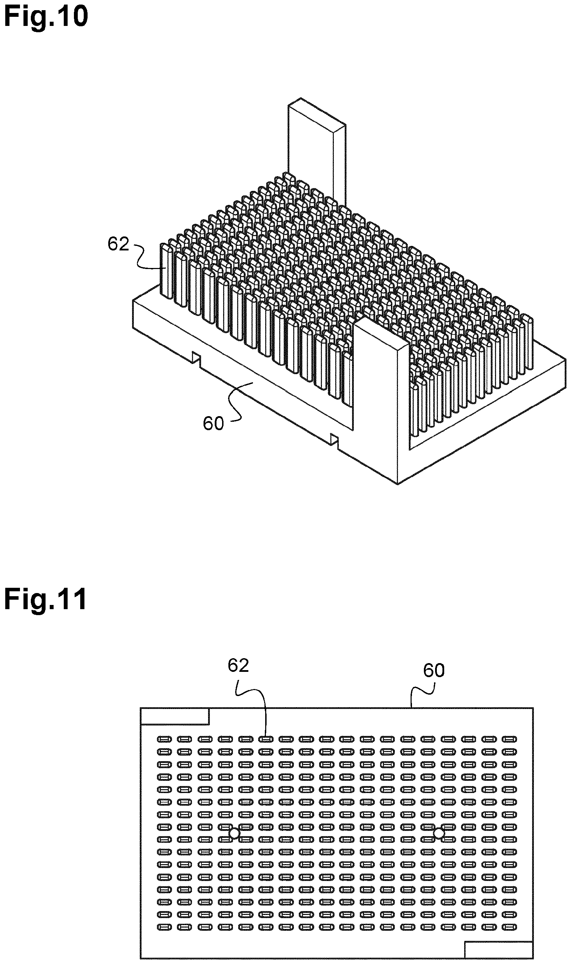

Les Figures 7 à 9 illustrent, respectivement en perspective, en élévation et de bout, l'une de ces deux pièces déformables 60 avec la capsule 10 reposant au centre, tandis que les Figures 10, 11 illustrent isolément, respectivement en perspective et en plan, la seule pièce déformable 60.

La pièce 60 se présente sous la forme d'un socle plat supportant une texture de brins ou lamelles 62, par exemple un ensemble réalisé monobloc par moulage ou injection d'un matériau flexible tel que polyéthylène, polypropylène, nylon, silicone ou polyuréthanne thermoplastique, de préférence avec une rigidité inférieure à 80 Shore A. Les brins ou lamelles 62 peuvent se présenter sous la forme de fils, fibres ou lames ; dans ce dernier cas, comme illustré sur les figures, la plus grande dimension de chaque lame est de préférence parallèle à l'axe principal de la capsule, de manière à favoriser la déformation en flexion : comme on peut le voir notamment Figure 9, les brins ou lamelles entrent en contact tangentiel avec la surface de la capsule 10, et la pression tangentielle exercée sur cette dernière peut éventuellement être ajustée en adaptant l'écartement d entre les deux pièces déformables 60, 60' en vis-à-vis.

Les brins ou lamelles souples déformables en flexion 62 peuvent, en variante, être remplacés par un bloc massif en mousse. Toutefois, l'utilisation d'une texture de brins ou lamelles permet de ménager entre ces brins ou lamelles un espace qui rend la texture latéralement perméable aux gaz : il est ainsi possible de faire circuler jusqu'à la capsule un gaz de stérilisation introduit depuis l'extérieur de la structure d'amortissement 58 même dans une configuration où la capsule est déjà enveloppée et calée dans la structure d'amortissement. L'élasticité, le dimensionnement et le nombre des brins ou lamelles sont par ailleurs avantageusement choisis de manière à assurer à l'encontre des vibrations un filtrage qui soit maximal pour les fréquences inférieures à 40 Hz environ, c'est-à-dire dans le domaine des vibrations les plus susceptibles d'endommager la lame piézoélectrique 24 par entrée en résonance de l'ensemble inertiel pendulaire.

Grâce à la structure d'amortissement 58, l'ensemble est ainsi protégé à l'encontre des chocs et vibrations dans les trois dimensions X, Y et Z.

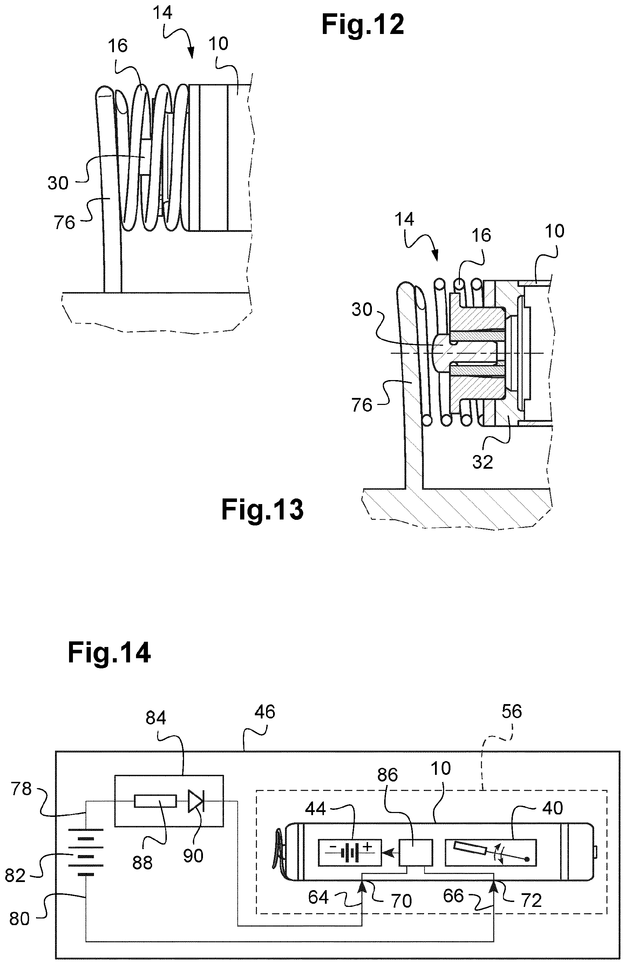

Comme illustré Figures 12 et 13, la pièce déformable 60 comprend avantageusement une butée frontale axialement déformable 76 permettant en particulier de protéger l'électrode de stimulation distale 30 afin qu'aucun élément ne puisse entrer en contact avec celle-ci. En effet, ce type d'électrode est habituellement recouvert d'un revêtement de type nitrure de titane de très faible épaisseur (moins de 10 µm), très performant sur le plan de la stimulation mais très fragile et sensible aux rayures et contaminations particulaires. La raideur axiale de la vis d'ancrage 16 est par ailleurs adaptée pour être supérieure à l'effort appliqué par une ou plusieurs butées axiales 76, typiquement supérieure à 1 N.

Si l'on revient à la Figure 5, la structure d'amortissement 58, formée des deux pièces déformables 60, 60' enfermant la capsule 10, est logée dans une pièce rigide 64, par exemple une pièce découpée dans une matière telle que polypropylène, polyéthylène ou autre puis pliée avec formation de butées pour le maintien en position de la structure d'amortissement 58. La pièce rigide 64 supporte également l'extrémité du cathéter 50 et le housing 52 à proximité de la capsule enfermée dans la structure d'amortissement 58.

Outre la protection mécanique, il est prévu d'établir un couplage électrique avec des électrodes de la capsule, de manière à pouvoir recharger en tant que de besoin la batterie tampon intégrée à la capsule, de la manière qui sera exposée plus bas en référence à la Figure 14.

Pour cela, il est prévu des pointes de touche 66, 68 (Figure 8) venant en contact avec des surfaces conductrices distinctes 70, 72 du corps tubulaire 12 de la capsule 10.

Une telle structure de corps tubulaire comprenant deux surfaces conductrices 70, 72 (métalliques) séparées par une surface cylindrique isolante 74 (céramique) est décrite par exemple dans le EP 3 730 185 A1 (Cairdac), auquel on pourra se référer pour plus de détails.

Les pointes de touche 66, 68 peuvent être des tiges avec une extrémité télescopique ou une bille rétractable venant en contact avec les surfaces conductrices 70, 72 ; en variante, le couplage électrique peut être réalisé par des lames souples ou des ressorts conducteurs, ou par tout autre moyen assurant la même fonction.

Comme illustré notamment Figure 5, les pointes de touche 66, 68 sont reliées par des conducteurs respectifs 78, 80 à une source d'énergie électrique 82, déportée par rapport au support 56 de protection et de calage de la capsule.

La source d'énergie électrique peut être notamment une pile "bâton" 1,5 V conventionnelle (comme illustré Figure 5) ou une pile "bouton" de moindre encombrement (comme dans la variante illustrée Figure 6), selon la capacité énergétique recherchée, essentiellement liée à la durée de stockage garantie, comme on l'expliquera plus bas. En variante, la source d'énergie électrique déportée peut être un récepteur d'énergie inductive, par exemple une boucle de recharge par induction disposée dans le volume interne 48 du conditionnement stérile de l'emballage 46 ; cette boucle est alors couplée à un émetteur d'énergie inductive situé à l'extérieur du conditionnement stérile 46.

On va maintenant décrire, en référence au schéma électrique de la Figure 14, la manière dont le niveau de charge de la batterie tampon 44 peut être maintenu à un niveau minimal satisfaisant malgré l'absence de recharge par le harvester 40. Les conducteurs 78, 80 et les pointes de touche 66, 68 assurent un couplage galvanique entre la capsule 10 et la source d'énergie électrique déportée 82 (ci-après désignée "pile" par souci de simplicité).

La tension nominale de la pile 82 est choisie de manière à être supérieure à la tension opérationnelle de la batterie tampon 44, par exemple une tension de pile de 1,5 à 9 V, typiquement de 5 à 6 V, pour une tension de batterie tampon variant typiquement entre 3 V et 4,2 V. Si la tension de la pile est inférieure à celle de la batterie tampon, on peut prévoir un circuit *boost* élévateur de tension, soit externe à la capsule, soit interne à celle-ci (par exemple un étage élévateur de tension au sein du PMU 42 (Figure 3)).

Le couplage de la pile 82 à la batterie tampon 44 comprend, outre les conducteurs 78, 80 et les pointes de touche 64, 66, un circuit 84 d'interfaçage entre la pile 82 et la capsule 10, et un circuit d'interfaçage 86 interne à la capsule permettant de coupler les surfaces externes conductrices 70, 72 à la batterie tampon 44.

Dans sa configuration la plus simple, le circuit 84 d'interfaçage pile/capsule comprend une résistance 88 de limitation du courant de recharge délivré par la pile, et une diode 90 permettant d'interrompre la recharge lorsque le niveau de tension de la batterie 44 atteint la valeur de la tension de la pile 82. Dans une variante plus élaborée, le circuit d'interfaçage 84 peut comprendre un circuit permettant de déterminer le niveau de tension de la batterie et de commander sélectivement la délivrance du courant de recharge, en interrompant l'alimentation de la batterie 44 par la pile 82 lorsque le niveau de charge dépasse un seuil haut prédéfini, et en rétablissant cette alimentation lorsque le niveau de charge a chuté jusqu'à un seuil bas prédéfini.

Par ailleurs, il peut être prévu un moyen de contrôle visuel, par exemple au moyen d'une LED (non représentée), du couplage correct entre la pile 82 et la capsule 10, c'est-à-dire de vérification du bon état de la fonctionnalité de recharge contrôlée de la batterie tampon de la capsule par la pile 82 à l'intérieur du conditionnement étanche.

Du point de vue quantitatif, pour un courant de veille et une autodécharge de la batterie entraînant un courant permanent de l'ordre de 1 µA et pour une capacité de la batterie de l'ordre de 1 mAh, on aboutit normalement à une longévité sur étagère de l'ordre de 1000 h, soit environ 40 jours, du fait de l'absence de recharge par le harvester, qui est immobile.

Pour garantir une durée de stockage de 24 mois pendant laquelle on veut être certain que la capsule reste fonctionnelle bien qu'en veille, il sera nécessaire de prévoir environ 30 cycles de recharge de la batterie 44 par la pile 82. Si l'on estime le rendement de l'opération à 50 %, ceci donne pour la pile externe 82 une capacité de 60 mAh, valeur tout à fait compatible avec ce que procurent les piles "bouton" conventionnelles, qui ont typiquement une capacité de l'ordre de 80 à 100 mAh ou plus.

L'invention permet ainsi, avec des moyens très simples, de garantir en toutes circonstances un stockage sur étagère de très longue durée, sans diminution aucune de la longévité de la capsule, celle-ci étant toujours fonctionnelle et prête à être réveillée à tout moment pour permettre son implantation.

## Revendications

1. Un accessoire pour le transport et le stockage d'un implant cardiaque autonome notamment de type capsule leadless (10) avant implantation, l'accessoire comprenant une source externe d'énergie électrique (82) pour le stockage temporaire d'une énergie électrique pendant le transport et le stockage de l'implant, la source externe (82) étant matériellement séparée de l'implant (10), et une liaison (66, 68, 78, 80, 84, 86) de couplage électrique temporaire de la source externe (82) à l'implant,
l'implant comprenant un corps tubulaire logeant :
- un module de récupération d'énergie (40) apte à convertir en énergie électrique des sollicitations externes appliquées à l'implant, au moyen d'un ensemble inertiel pendulaire comprenant un élément élastiquement déformable (24) couplé à une masse inertielle (26) ; et
- une batterie rechargeable (44) apte à être rechargée par le module de récupération d'énergie (40), la batterie étant préalablement chargée à un niveau de charge initial,
la liaison (66, 68, 78, 80, 84, 86) de couplage électrique est une liaison à la batterie rechargeable (44) de l'implant, de manière à assurer une alimentation de la batterie rechargeable par la source externe et ainsi maintenir, pendant toute la durée du transport et du stockage avant implantation, un niveau de charge de la batterie (44) supérieur à un niveau minimal prédéterminé ; et
l'accessoire comprend en outre :
- un support de protection (56) apte à recevoir et caler l'implant (10) par rapport à l'accessoire dans une configuration assurant le couplage électrique de l'implant à la source externe, dans lequel le support de protection (56) comprend une structure (58) d'amortissement de chocs et de filtrage de vibrations comprenant une texture de brins ou lamelles (62) élastiquement déformables enveloppant et calant en position l'implant (10) à l'intérieur du support de protection (56).

2. L'accessoire de la revendication 1, dans lequel le support de protection (56) comprend en outre des bornes électriques (64, 66) reliées à la source externe (82) et aptes à venir en contact avec des électrodes de surface respectives du corps tubulaire de l'implant (10), les électrodes de surface étant couplées (86) à l'intérieur de l'implant à la batterie rechargeable (44).

3. L'accessoire de la revendication 2, dans lequel les bornes électriques comprennent des pointes de touche (64, 66) rétractables faisant saillie à l'intérieur du support de protection et aptes à venir en appui radial contre les électrodes de surface respectives (70, 72) du corps tubulaire de l'implant.

4. L'accessoire de la revendication 1, dans lequel les brins ou lamelles (62) élastiquement déformables sont aptes, en configuration déformée au contact de l'implant, à venir en contact tangentiel avec la surface du corps tubulaire de l'implant.

5. L'accessoire de la revendication 1, dans lequel la texture de brins ou lamelles (62) est latéralement perméable aux gaz, de manière à permettre, dans une configuration où l'implant (10) est enveloppé et calé dans la structure d'amortissement, la circulation jusqu'à l'implant d'un gaz de stérilisation introduit depuis l'extérieur de la structure d'amortissement.

6. L'accessoire de la revendication 1, dans lequel le support de protection comprend en outre une butée axiale (76) élastiquement déformable apte à venir en contact avec une extrémité frontale (14) et/ou postérieure (18) du corps tubulaire de l'implant.

7. L'accessoire de la revendication 1, comprenant en outre un composant (88) de limitation du courant délivré par la source externe (82) à la batterie rechargeable (44) de l'implant par l'intermédiaire de la liaison de couplage électrique temporaire.

8. L'accessoire de la revendication 1, comprenant en outre un témoin de contrôle du couplage et/ou du passage d'un courant de la source externe à la batterie rechargeable de l'implant par l'intermédiaire de la liaison de couplage électrique temporaire.

9. L'accessoire de la revendication 1, comprenant en outre un circuit d'évaluation du niveau de charge de la batterie, et un circuit apte à interrompre l'alimentation de la batterie rechargeable par la source externe lorsque le niveau de charge évalué dépasse un seuil haut prédéfini, et à rétablir l'alimentation de la batterie rechargeable par la source externe lorsque le niveau de charge évalué atteint un seuil bas prédéfini.

10. Un conditionnement pour le transport et le stockage d'un implant cardiaque autonome notamment de type capsule leadless (10) avant implantation, comprenant :
- un emballage étanche (46) définissant un volume interne stérile (48) enfermant l'implant (10), l'implant comprenant un corps tubulaire logeant :
· un module de récupération d'énergie (40) apte à convertir en énergie électrique des sollicitations externes appliquées à l'implant, au moyen d'un ensemble inertiel pendulaire comprenant un élément élastiquement déformable (24) couplé à une masse inertielle (26), et
· une batterie rechargeable (44) apte à être rechargée par le module de récupération d'énergie (40), la batterie étant préalablement chargée à un niveau de charge initial,
le conditionnement comprenant en outre, à l'intérieur du volume stérile (48), un accessoire selon la revendication 1.

11. Le conditionnement de la revendication 10, dans lequel la source externe (82) est une pile électrique non rechargeable logée à l'intérieur de l'emballage étanche.

12. Le conditionnement de la revendication 10, dans lequel la source externe est un récepteur d'énergie inductive logé à l'intérieur de l'emballage étanche et couplé par voie non galvanique à un émetteur d'énergie inductive extérieur à l'emballage étanche.

## Patentansprüche

1. Zubehörteil für den Transport und die Lagerung eines autonomen Herzimplantats insbesondere vom Typ drahtlose Kapsel (10) vor Implantation,
wobei das Zubehörteil eine externe Quelle elektrischer Energie (82) für die vorübergehende Speicherung einer elektrischen Energie während des Transports und der Lagerung des Implantats umfasst, wobei die externe Quelle (82) von dem Implantat (10) materiell getrennt ist, und eine Verbindung (66, 68, 78, 80, 84, 86) zur vorübergehenden elektrischen Kopplung der externen Quelle (82) mit dem Implantat umfasst,
wobei das Implantat einen rohrförmigen Körper umfasst, der Folgendes aufnimmt:
- ein Energierückgewinnungsmodul (40), das imstande ist, äußere Belastungen, die auf das Implantat aufgebracht werden, in elektrische Energie umzuwandeln, mittels einer pendelartigen inertialen Anordnung, die ein elastisch verformbares Element (24) umfasst, das mit einer trägen Masse (26) gekoppelt ist; und
- eine wiederaufladbare Batterie (44), die imstande ist, durch das Energierückgewinnungsmodul (40) wiederaufgeladen zu werden, wobei die Batterie zuvor auf einen anfänglichen Ladezustand geladen wird,
wobei die Verbindung (66, 68, 78, 80, 84, 86) für die elektrische Kopplung ist eine Verbindung der wiederaufladbaren Batterie (44) mit dem Implantat, derart, dass eine Versorgung der wiederaufladbaren Batterie durch die externe Quelle sichergestellt wird und so, während der gesamten Dauer des Transports und der Lagerung vor Implantation, ein Ladezustand der Batterie (44) aufrecht erhalten wird, der höher ist als ein vorbestimmter minimaler Zustand; und das Zubehörteil ferner umfasst:
- einen Schutzträger (56), der imstande ist, das Implantat (10) aufzunehmen und relativ zu dem Zubehörteil in einer Konfiguration zu blockieren, welche die elektrische Kopplung des Implantats mit der externen Quelle sicherstellt, wobei der Schutzträger (56) eine Struktur (58) zur Dämpfung von Stößen und zur Filterung von Vibrationen umfasst, die eine Textur aus elastisch verformbaren Fäden oder Blättchen (62) umfasst, die das Implantat (10) umhüllen und in seiner Position im Inneren des Schutzträgers (56) blockieren.

2. Zubehörteil nach Anspruch 1, wobei der Schutzträger (56) ferner elektrische Klemmen (64, 66) umfasst, die mit der externen Quelle (82) verbunden sind und imstande sind, mit jeweiligen Oberflächenelektroden des rohrförmigen Körpers des Implantats (10) in Kontakt zu kommen, wobei die Oberflächenelektroden im Inneren des Implantats mit der wiederaufladbaren Batterie (44) gekoppelt (86) sind.

3. Zubehörteil nach Anspruch 2, wobei die elektrischen Klemmen (64, 66) einziehbare Kontaktpunkte (64, 66) umfassen, die in das Innere des Schutzträgers vorspringen und imstande sind, gegen die jeweiligen Oberflächenelektroden (70, 72) des rohrförmigen Körpers des Implantats in radiale Abstützung zu kommen.

4. Zubehörteil nach Anspruch 1, wobei die elastisch verformbaren Fäden oder Blättchen (62) imstande sind, in beim Kontakt mit dem Implantat verformter Konfiguration, mit der Oberfläche des rohrförmigen Körpers des Implantats in tangentialen Kontakt zu kommen.

5. Zubehörteil nach Anspruch 1, wobei die Textur aus Fäden oder Blättchen (62) seitlich für Gase durchlässig ist, derart, dass sie, in einer Konfiguration, wo das Implantat (10) umhüllt und in der Dämpfungsstruktur blockiert ist, die Zirkulation eines Sterilisationsgases, das von außerhalb der Dämpfungsstruktur eingeleitet wird, bis zu dem Implantat hin gestattet.

6. Zubehörteil nach Anspruch 1, wobei der Schutzträger ferner einen elastisch verformbaren axialen Anschlag (76) umfasst, der imstande ist, mit einem frontalen Ende (14) und/oder hinteren Ende (18) des rohrförmigen Körpers des Implantats in Kontakt zu kommen.

7. Zubehörteil nach Anspruch 1, ferner umfassend ein Bauteil (88) zur Begrenzung des von der externen Quelle (82) über die Verbindung zur vorübergehenden elektrischen Kopplung an die wiederaufladbare Batterie (44) des Implantats gelieferten Stroms.

8. Zubehörteil nach Anspruch 1, ferner umfassend eine Leuchte zum Überprüfen der Kopplung und/oder des Durchgangs eines von der externen Quelle über die Verbindung zur vorübergehenden elektrischen Kopplung an die wiederaufladbare Batterie des Implantats gelieferten Stroms.

9. Zubehörteil nach Anspruch 1, ferner umfassend eine Schaltung zur Auswertung des Ladezustands der Batterie und eine Schaltung, die imstande ist, die Versorgung der wiederaufladbaren Batterie durch die externe Quelle zu unterbrechen, wenn der ausgewertete Ladezustand einen vordefinierten oberen Schwellwert übersteigt, und die Versorgung der wiederaufladbaren Batterie durch die externe Quelle wiederherzustellen, wenn der ausgewertete Ladezustand einen vordefinierten unteren Schwellwert erreicht.

10. Verpackung für den Transport und die Lagerung eines autonomen Herzimplantats insbesondere vom Typ drahtlose Kapsel (10) vor Implantation, umfassend:
- eine dichte Umhüllung (46), die ein steriles Innenvolumen (48) definiert, welches das Implantat (10) umschließt, wobei das Implantat einen rohrförmigen Körper umfasst, der Folgendes aufnimmt:
• ein Energierückgewinnungsmodul (40), das imstande ist, äußere Belastungen, die auf das Implantat aufgebracht werden, in elektrische Energie umzuwandeln, mittels einer pendelartigen inertialen Anordnung, die ein elastisch verformbares Element (24) umfasst, das mit einer trägen Masse (26) gekoppelt ist; und
• eine wiederaufladbare Batterie (44), die imstande ist, durch das Energierückgewinnungsmodul (40) wiederaufgeladen zu werden, wobei die Batterie zuvor auf einen anfänglichen Ladezustand geladen wird,
wobei die Verpackung ferner, im Inneren des sterilen Innenvolumens (48), ein Zubehörteil nach Anspruch 1 umfasst.

11. Verpackung nach Anspruch 10, wobei die externe Quelle (82) eine nicht wiederaufladbare Batterie ist, die im Inneren der dichten Umhüllung aufgenommen ist.

12. Verpackung nach Anspruch 10, wobei die externe Quelle ein induktiver Stromempfänger ist, der im Inneren der dichten Umhüllung aufgenommen ist und auf nicht galvanischem Wege mit einem induktiven Stromsender außerhalb der dichten Umhüllung gekoppelt ist.

## Claims

1. An accessory for transportation and storage of an autonomous cardiac implant, in particular of the leadless capsule (10) type, before implantation,
the accessory comprising an external source of electrical energy (82) for the temporary storage of an electrical energy during the transportation and storage of the implant, the external source (82) being physically separated from the implant (10), and a temporary electrical coupling link (66, 68, 78, 80, 84, 86) from the external source (82) to the implant,
wherein the implant comprises a tubular body which houses:
- an energy harvesting module (40) adapted to convert external stresses applied to the implant into electrical energy, by means of an inertial pendular unit comprising an elastically deformable element (24) coupled to an inertial mass (26); and
- a rechargeable battery (44) adapted to be recharged by the energy harvesting module (40), the battery being previously charged at an initial charge level,
the electrical coupling link (66, 68, 78, 80, 84, 86) is a link to the implant rechargeable battery (44), in such a way as to ensure a power supply of the rechargeable battery by the external source and hence to maintain, during the whole transportation and storage duration before implantation, a charge level of the battery (44) higher than a minimum predetermined level; and
the accessory further comprises:
- a protection support (56) for receiving and wedging the implant (10) with respect to the accessory in a configuration ensuring the electrical coupling of the implant to the external source, wherein the protection support (56) comprises a shock-absorbing and vibration-filtering structure (58) comprising a texture of elastically deformable strands or slats (62) wrapping and wedging the implant(10) in position inside the protection support (56).

2. The accessory according to claim 1, wherein the protection support (56) further comprises electrical terminals (64, 66) connected to the external source (82) and adapted to come into contact with respective surface electrodes of the tubular body of the implant (10), the surface electrodes being coupled (86), inside of the implant, to the rechargeable battery (44).

3. The accessory according to claim 2, wherein the electrical terminals comprise retractable touch tips (64, 66) protruding inside the protection support and adapted to come into radial contact against the respective surface electrodes (70, 72) of the implant tubular body.

4. The accessory according to claim 1, wherein the elastically deformable strands or slats (62) are adapted, in a deformed configuration in contact with the implant, to come into tangential contact with the surface of the implant tubular body.

5. The accessory according to claim 1, wherein the texture of the strands or slats (62) is laterally permeable to gas, in such a way as to allow, in a configuration in which the implant (10) is wrapped and wedged in the absorbing structure, a circulation up to the implant of a sterilization gas introduced from the outside of the absorbing structure.

6. The accessory according to claim 1, wherein the protection support further comprises an elastically deformable axial stop (76) adapted to come into contact with a front (14) and/or back (18) end of the implant tubular body.

7. The accessory according to claim 1, further comprising a component (88) for limiting the current delivered by the external source (82) to the implant rechargeable battery (44) through the temporary electrical coupling link.

8. The accessory according to claim 1, further comprising a control indicator for the coupling and/or the passage of a current from the external source to the implant rechargeable battery through the temporary electrical coupling link.

9. The accessory according to claim 1, further comprising a circuit for evaluating the battery charge level, and a circuit adapted to interrupt the power supply of the rechargeable battery by the external source when an evaluated charge level exceeds a predefined high threshold, and to reestablish the power supply of the rechargeable battery by the external source when the evaluated charge level reaches a predefined low threshold.

10. A packaging for the transportation and storage of an autonomous cardiac implant, in particular of the leadless capsule (10) type, before implantation, comprising:
- a sealed package (46) defining a sterile internal volume (48) enclosing the implant, wherein the implant comprises a tubular body which houses:
• an energy harvesting module (40) adapted to convert external stresses applied to the implant into electrical energy, by means of an inertial pendular unit comprising an elastically deformable element (24) coupled to an inertial mass (26), and
• a rechargeable battery (44) adapted to be recharged by the energy harvesting module (40), the battery being previously charged at an initial charge level,
wherein the packaging further comprises, inside the sterile internal volume (48), an accessory according to claim 1.

11. The packaging of claim 10, wherein the external source (82) is a non-rechargeable electric cell housed inside the sealed package.

12. The package of claim 10, wherein the external source is an inductive energy receiver housed inside the sealed package and non-galvanically coupled to an inductive energy emitter external to the sealed package.
